# EUROPEAN PATENT SPECIFICATION

(11) **EP 2 794 617 B1**
(45) Date of publication and mention of the grant of the patent: **09.03.2016**
(21) Application number: 12808371.4
(22) Date of filing: 19.12.2012
(51) Int. Cl.: C07D 495/04

(54) **PROCESS FOR THE PREPARATION OF BENZODITHIOPHENE COMPOUNDS**
VERFAHREN ZUR HERSTELLUNG VON BENZODITHIOPHENVERBINDUNGEN
PROCÉDÉ DE PRÉPARATION DE COMPOSÉS DE BENZODITHIOPHÈNE

(30) Priority: 19.12.2011 IT MI20112303
(43) Date of publication of application: 29.10.2014
(73) Proprietor: ENI S.p.A., 00144 Roma (IT)
(72) Inventor: BIANCHI, Gabriele, I-28100 Novara (IT); SCHIMPERNA, Giuliana, I-28100 Novara (IT)
(74) Representative: Mauro, Marina Eliana
(86) International application number: PCT/EP2012/076063
(87) International publication number: WO 2013/092648

(56) References cited:
- WATANABE H ET AL: "Synthesis of Alkylated Benzo(2,1-b:3,4-b') dithiophenes by Annulative Coupling and Their Direct Arylation under Palladium Catalysis", CHEMISTRY LETTERS, vol. 36, no. 11, 1 November 2007 (2007-11-01), pages 1336-1337, XP009113911, ISSN: 0366-7022, DOI: 10.1246/CL.2007.1336 [retrieved on 2007-10-13] cited in the application
- MASAKI SHIMIZU ET AL: "Palladium-catalyzed double cross-coupling reaction of 1,2-bis(pinacolatoboryl)alkenes and -arenes with 2,2-dibromobiaryls: annulative approach to functionalized polycyclic aromatic hydrocarbons", TETRAHEDRON, ELSEVIER SCIENCE PUBLISHERS, AMSTERDAM, NL, vol. 67, no. 41, 6 August 2011 (2011-08-06), pages 8014-8026, XP028288356, ISSN: 0040-4020, DOI: 10.1016/J.TET.2011.08.019 [retrieved on 2011-08-12]
- ANDRZEJ RAJCA ET AL: "Functionalized Thiophene-Based [7]Helicene: Chirooptical Properties versus Electron Delocalization", THE JOURNAL OF ORGANIC CHEMISTRY, vol. 74, no. 19, 2 October 2009 (2009-10-02), pages 7504-7513, XP055029517, ISSN: 0022-3263, DOI: 10.1021/jo901769c
- LAROCK RICHARD C ET AL: "Synthesis of Polycyclic Aromatic Hydrocarbons by Pd-Catalyzed Annulation of Alkynes", JOURNAL OF ORGANIC CHEMISTRY, AMERICAN CHEMICAL SOCIETY, EASTON.; US, vol. 62, no. 22, 1 January 1997 (1997-01-01), pages 7536-7537, XP002493797, ISSN: 0022-3263, DOI: 10.1021/JO9712557

## Description

The present invention relates to a process for the preparation of a benzodithiophene compound.

More specifically, the present invention relates to a process for the preparation of a benzodithiophene compound which comprises reacting at least one monohalogenated dithiophene compound with at least one internal alkyne.

Said benzodithiophene compound, after suitable functionalization and polymerization, can be advantageously used in the construction of photovoltaic devices such as, for example, photovoltaic cells, photovoltaic modules, solar cells, solar modules, on both rigid and flexible supports. Furthermore said benzodithiophene compound can be advantageously used as spectrum converter in luminescent solar concentrators (LSC). Said benzodithiophene compound can also be advantageously used as precursor of monomeric units in the preparation of semiconductor polymers.

Photovoltaic devices are capable of converting the energy of a light radiation into electric energy. At present, most photovoltaic devices which can be used for practical applications exploit the physico-chemical properties of photoactive materials of the inorganic type, in particular high-purity crystalline silicon. As a result of the high production costs of silicon, scientific research, however, has been orienting its efforts towards the development of alternative organic materials having a conjugated, oligomeric or polymeric structure, in order to obtain organic photovoltaic devices such as, for example, organic photovoltaic cells. Unlike high-purity crystalline silicon, in fact, these materials of an organic nature are characterized by a relative synthesis facility, a low production cost, a reduced weight of the relative photovoltaic devices, and also allow the recycling of said materials of the organic type at the end of the life cycle of the organic photovoltaic device in which they are used.

The advantages indicated above make the use of these materials of the organic type energetically and economically interesting in spite of possible lower efficiencies (η) of the organic photovoltaic devices thus obtained with respect to inorganic photovoltaic devices.

The functioning of organic photovoltaic devices such as, for example, organic photovoltaic cells, is based on the combined use of an electron-acceptor compound and of an electron-donor compound. In the state of the art, the most widely used electron-acceptor compounds in organic photovoltaic devices are fullerene derivatives, in particular PC61BM(6,6-phenyl-C₆₁-butyric acid methyl ester) or PC71BM (6,6-phenyl-C₇₁-butyric acid methyl ester), which have reached the greatest efficiencies when mixed with electron-donor compounds selected from π-conjugated polymers such as, for example, polythiophenes (η > 5%), polycarbazoles (η > 6%), derivatives of poly(thienothiophene)-benzodithiophene (PTB) (η > 8%).

The basic conversion process of light into electric current in an organic photovoltaic cell takes place through the following steps:
1. absorption of a photon on the part of the electron-donor compound with the formation of an exciton, i.e. a pair of "electron-electronic gap (or hole)" charge transporters;
2. diffusion of the exciton in a region of the electron-donor compound as far as the interface with the electron-acceptor compound;
3. dissociation of the exciton in the two charge transporters: (electron (-) in the acceptor phase (i.e. in the electron-acceptor compound) and electronic gap (or hole) (+)) in the donor phase (i.e. in the electron-donor compound);
4. transporting of the charges thus formed to the cathode (electron, through the electron-acceptor compound) and to the anode [electronic gap (or hole), through the electron-donor compound], with the generation of an electric current in the circuit of the organic photovoltaic cell.

The photo-absorption process with the formation of the exciton and subsequent yielding of the electron to the electron-acceptor compound leads to the excitation of an electron from the HOMO (Highest Occupied Molecular Orbital) to the LUMO (Lowest Unoccupied Molecular Orbital) of the electron-donor compound, and subsequently, the passage from this to the LUMO of the electron-acceptor compound.

As the efficiency of an organic photovoltaic cell depends on the number of free electrons that are generated by dissociation of the excitons which, in their turn, can be directly correlated with the number of photons absorbed, one of the structural characteristics of electron-donor compounds which mostly influences said efficiency is the difference in energy existing between the HOMO and LUMO orbitals of the electron-donor compound, or the so-called band-gap. In particular, the maximum wave-length value at which the electron-donor compound is capable of collecting and effectively converting photons into electric energy, i.e. the so-called "photon harvesting" or "light-harvesting" process, depends on this difference. In order to obtain acceptable electric currents, the band-gap, i.e. the difference in energy between HOMO and LUMO of the donor compound, must not be excessively high to allow the absorption of the highest number of photons, but at the same time not excessively low as it could reduce the voltage at the electrodes of the device.

In the simplest way of operating, organic photovoltaic cells are produced by introducing a thin layer (about 100 nanometres) of a mixture of the electron-acceptor compound and of the electron-donor compound (architecture known as "bulk heterojunction"), between two electrodes, normally consisting of indium-tin oxide (ITO) (anode) and aluminium (Al) (cathode). In order to produce a layer of this type, a solution of the two compounds is generally prepared and a photoactive film is subsequently created on the anode [indium-tin oxide (ITO] starting from this solution, resorting to suitable deposition techniques such as, for example, "spin-coating", "spray-coating" "ink-jet printing", and the like. Finally, the counter-electrode [i.e. the aluminium cathode (Al)] is deposited on the dried film. Optionally, other additional layers capable of exerting specific functions of an electric, optical or mechanical nature, can be introduced between the electrodes and the photoactive film.

In order to facilitate the electron gaps (or holes) in reaching the anode [indium-tin oxide (ITO)] and at the same time in blocking the transporting of electrons, thus allowing an improved collection of the charges on the part of the electrode and inhibiting recombination phenomena, before creating the photoactive film, starting from the mixture of acceptor compound and of donor compound as described above, a film is deposited, starting from an aqueous suspension of PEDOT:PSS [poly(3,4-ethylenedioxythiophene)-polystyrene sulfonate], resorting to suitable deposition techniques such as, for example, "spin-coating", "spray-coating" "ink-jet printing", and the like.

The electron-donor compound which is most commonly used in the construction of organic photovoltaic cells is regioregular poly(3-hexylthiophene) (P3HT). This polymer has optimal electronic and optical characteristics (good HOMO and LUMO orbital values, good molar adsorption coefficient), a good solubility in the solvents used in the construction of photovoltaic cells and a reasonable mobility of the electronic holes.

Other examples of polymers which can be advantageously used as electron-donor compounds are: the polymer PCDTBT {poly[N-9"-heptadecanyl-2,7-carbazole-alt-5,5-(4',7'-di-2-thienyl-2',1',3'-benzothiadiazole]}, the polymer PCPDTBT {poly[2,6-(4,4-bis-(2-ethylhexyl)-4*H*-cyclopenta[2,1-b;3,4-b']-dithiophene)-alt-4,7-(2,1,3-benzothiadiazole)]}.

Electron-donor compounds containing benzodithiophene units having a structure similar to poly(3-hexylthiophene) (P3HT) are also known, wherein the thiophene units, however, are planarized by means of benzene rings. This characteristic not only reduces the oxidation potential of said electron-donor compounds but also improves their stability to air and guarantees their rapid packing and, consequently, a high molecular order, during the formation of the photoactive film: this leads to excellent charge transporting properties [electrons or electronic gaps (holes)]. The use of electron-donor compounds containing benzodithiophene units therefore enables the production of photovoltaic devices having improved performances.

Electron-donor compounds containing benzodithiophene units are described, for example, by Huo L. et al. in the article: "Synthesis of a polythieno[3,4-b]thiophene derivative with a low-lying HOMO level and its application in polymer solar cells", "Chemical Communication" (2011), Vol. 47, pages 8850-8852. This article describes the preparation of a polythieno[3,4-b]thiophene derivative by copolymerization between a planar benzodithiophene having a low HOMO value with a thieno[3,4-b]thiophene unit.

It is known that benzodithiophene and/or its isomers [e.g., benzo[1,2-*b*:4,5-*b*']dithiophene or (BDT) and benzo[2,1-*b:*3,4-*b*']dithiophene or (BDP)], are compounds of great interest whose synthesis has been the object of a lot of research.

Benzodithiophene and/or its isomers can generally be prepared by means of three different processes.

A first process comprises an annulation reaction known as McMurry reaction, of a diketone-2,2'-dithiophene. This annulation reaction is generally carried out in the presence of catalysts containing titanium and zinc, at a temperature ranging from 60°C to 80°C, in the presence of solvents such as, for example, tetrahydrofuran (THF), dioxane, for a time ranging from 8 hours to 12 hours. The yields of benzodithiophene and/or of its isomers generally range from 30% to 90%.

Further details relating to said first process can be found, for example, in the article of Yoshida S. et al.: "Novel Electron Acceptors Bearing a Heteroquinonoid System. 4. Syntheses, Properties, and Charge-Transfer Complexes of 2,7-Bis(dicyanomethylene)-2,7-dihydro-benzo[2,1-b:3,4-b']dithiophene, 2,7-Bis(dicyano-methylene)-2,7-dihydrobenzo-[1,2-b:4,3-b']-dithiophene, and 2,6-Bis(dicyanomethylene)-2,6-dihydrobenzo-[1,2-b:4,5-b']-dithiophene", "Journal of Organic Chemistry" (1994), Vol. 59, No. 11, pages 3077-3081. In said article, it is disclosed the preparation of a dicyanoalkylene-benzodithiophene starting from benzodithiophene isomers such as, for example, benzo[2,1-*b*:3,4-*b*']-dithiophene, benzo[1,2-*b*:4,3-*b*']-dithiophene, benzo[1,2-*b*:4,5-*b*']-dithiophene. Said benzodithiophene isomers can be obtained by the reaction of 2,2'-dithiophene-3,3'-dicarboxaldeyde with titanium tetrachloride (TiCl₄) and zinc (Zn) metal, in the presence of anhydrous tetrahydrofuran.

Further details relating to this first process can also be found in the article of Rajca S. et al.: "Functionalized Thiophene-Based [7]Helicene: Chirooptical Properties versus Electron Delocalization", "Journal of Organic Chemistry" (2009), Vol. 74, No. 19, pages 7504-7513. In said article, it is disclosed the preparation of an enantiomerically pure functionalized [7]helicene, deriving from a di(benzodithiophene) functionalized with four heptyl groups. The preparation of a benzodithiophene is also described, by the reaction of a 3,4-dibromothiophene with lithium diisopropylamide (LDA) to give a dilithiate derivative which is subsequently reacted with N-methoxy-N-methyloctanamide to give the corresponding diketone. Said diketone is subsequently reacted with titanium tetrachloride (TiCl₄) and zinc (Zn) metal obtaining benzodithiophene.

The second process provides an annulation reaction between a diiodio-dithiophene and an excess of internal alkyne. This reaction is generally carried out in the presence of catalysts containing palladium, at a temperature ranging from 120°C to 140°C, in the presence of solvents such as, for example, *N,N-*dimethylformamide (DMF), toluene, o-xylene, for a time ranging from 4 hours to 48 hours. The yields generally range from 50% to 90%.

Further details relating to this second process can be found, for example, in the article of Watanabe H. et al.: "Synthesis of Alkylated Benzo[2,1-b:3,4-b']dithiophenes by Annulative Coupling and Their Direct Arylation under Palladium Catalysis","Chemistry Letters" (2007), Vol. 36, No. 11, pages 1336-1337. In said article, it is disclosed the preparation of a dialkyl derivative of benzo[2,1-*b*:3,4-*b*']dithiophene by the reaction of 3,3'-diiodo-2,2'-dithiophene with 4-octine, in the presence of *N,N*-dimethylformamide (DMF) and palladium(II) acetate Pd(OAc)₂ and *N*-methyl-dicyclohexylamine as catalyst.

The third process provides an annulation reaction between a dibromo-dithiophene and a vic-bis-(pinacolatoboryl)alkene or a *vic*-bis(pinacolatoboryl)-phenanthrene. This reaction is generally carried out in the presence of catalysts containing palladium, at a temperature ranging from 60°C to 80°C, in the presence of solvents such as, for example, tetrahydrofuran (THF), toluene, for a time ranging from 24 hours to 48 hours. The yields generally range from 50% to 90%.

Further details relating to this third process can be found, for example, in the article of Shimizu M. et al.: "Palladium-Catalyzed Annulation of vic-Bis(pinacolatoboryl)alkenes and -phenanthrenes with 2,2'-dibromobiaryls: Facile Synthesis of Functionalized Phenanthrenes and Dibenzo[g,p]-chrysenes", "Angewandte Chemie International Edition" (2008), Vol. 47, pages 8096-8099. This article describes the preparation of a dialkyl-benzodithiophene by the reaction of a dibromo-dithiophene with a *vic*-bis(pinacolatoboryl)alkene in tetrahydrofuran (THF), in the presence of potassium carbonate (K₂CO₃) and of tetrakis(triphenylphosphine)-palladium(0) [Pd(PPh₃)₄] as catalyst.

Although the above processes allow benzodithiophene and/or its isomers to be obtained with good yields, generally higher than or equal to 50%, they can, however, have various disadvantages. In particular:
- the synthesis steps for obtaining the desired final compound are numerous;
- corrosive and/or flammable reagents are often used, such as, for example, titanium tetrachloride, lithium diisopropylamide (LDA), with consequent problems relating to the safety of both the environment and of the operators, with consequently higher costs for both production and disposal of the waste products.
- dihalogenated starting compounds are often used, such as, for example, diiodo-dithiophene or dibromo-dithiophene, which are generally costly and not particularly stable.

Processes for the preparation of polycyclic aromatic compounds through annulation reactions of aryl halides with internal alkynes, in the presence of palladium compounds as catalysts, are also known in the art.

Larock R. C. et al., for example, in the article: "Synthesis of Polycyclic Aromatic Hydrocarbons by the Pd-Catalyzed Annulation of Alkynes", "Journal of Organic Chemistry" (1997), Vol. 62, No. 22, pages 7536-7537, describe an annulation reaction with internal alkynes according to the following Scheme 1: 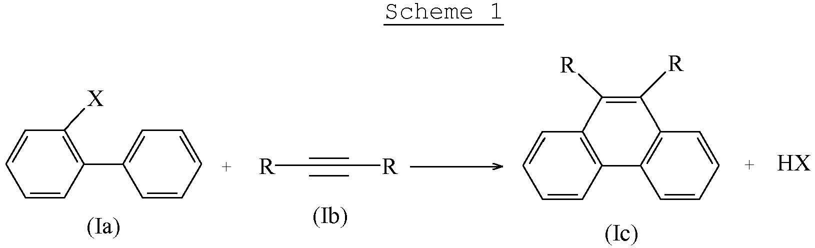 wherein an aryl halide having formula (Ia) such as, for example, 2-iodo-biphenyl, is reacted with an internal alkyne having formula (Ib) such as, for example, diphenylacetylene, in the presence of a catalyst containing palladium such as, for example, palladium(II)acetate ([Pd(OAc)₂]), a solvent such as, for example, dimethylformamide (DMF), and a base such as, for example, sodium acetate (NaOAc), obtaining a disubstituted phenanthrene having formula (Ic).

Huang H. et al., in the article "Palladium-catalyzed three-component domino reaction for the preparation of benzo[b]thiophene and related compounds", "Organic and Biomolecular Chemistry" (2011), Vol. 9, pages 5036-5038, describe a three-component domino annulation reaction, according to the following Scheme 2: 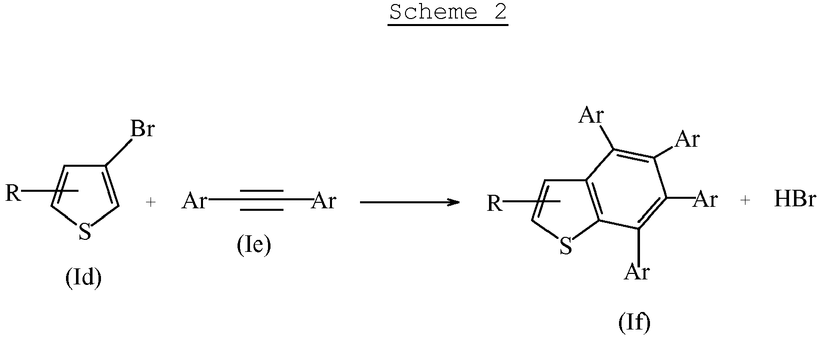 wherein a bromothiophene having formula (Id) such as, for example, 3-bromothiophene, is reacted with an internal alkyne having formula (Ie) such as, for example, diphenylacetylene, in the presence of a catalyst containing palladium such as, for example, palladium(II)acetate ([Pd(OAc)₂]), a phosphine such as, for example, tricyclohexylphosphine (PCy₃), a solvent such as, for example, dimethylformamide (DMF), and a base such as, for example, sodium carbonate (Na₂CO₃), obtaining a tetra-aryl-benzoalkyl-thiophene having formula (If).

Gericke K. M. et al., in the article: "The versatile role of norbornene in C-H functionalization processes: concise synthesis of tertracyclic fused pyrroles via a threefold domino reaction", "Tetrahedron" (2008), Vol. 64, pg. 6002-6014, describe an annulation reaction according to the following Scheme 3: 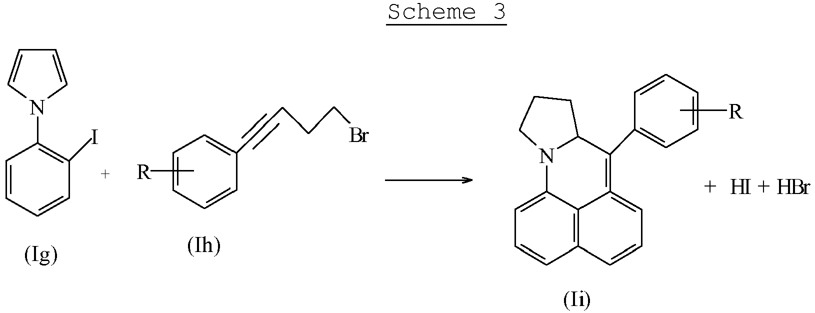 wherein an aryl iodide such as, for example, 1,2-iodophenyl-1-*H*-pyrrole having formula (Ig), is reacted with an internal bromo-alkylarylalkyne having formula (Ih) such as, for example, (5-bromo-1-pentenyl)benzene, in the presence of a catalyst containing palladium such as, for example, palladium(II)chloride (PdCl₂) associated with triphenylphosphine (PPh₃) as ligand, in the presence of a solvent such as, for example, acetonitrile (CH₃CN), and a base such as, for example, caesium carbonate (Cs₂CO₃), obtaining a 7-phenyl-5,6-dihydro-4H-benzo[de]pyrrole[1,2-α]-quinoline having formula (Ii).

No process is described in literature, however, which uses the annulation reaction for the synthesis of benzodithiophene derivatives starting from a monohalogenated dithiophene compound.

The Applicant has therefore considered the problem of finding a process for the preparation of a benzodithiophene compound capable of overcoming the drawbacks indicated above. In particular, the Applicant has considered the problem of finding a process for the preparation of a benzodithiophene compound by means of an annulation reaction starting from a monohalogenated dithiophene compound.

The Applicant has now found that the preparation of a benzodithiophene compound can be advantageously carried out by means of a process which comprises reacting at least one monohalogenated dithiophene compound with at least one internal alkyne.

There are numerous advantages obtained by operating according to the above process such as, for example:
- reduction of the number of synthesis steps with a relative reduction in the processing times and in the process costs;
- use of monohalogenated starting products generally more economical and more stable than the corresponding dihalogenated compounds;
- use of more economical and more stable internal alkynes with respect to diboron esters of internal alkenes;
- greater safety conditions [e.g., absence of corrosive and/or flammable reagents such as, for example, titanium tetrachloride, lithium diisopropylamide (LDA)] for both the health of the operators and from an environmental point of view;
- relatively short reaction temperatures and times thus avoiding the possible degradation of the product obtained and higher process costs.

An object of the present invention therefore relates to a process for the preparation of a benzodithiophene compound having general formula: 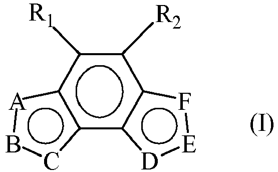 wherein:
- A, B, C, D, E and F, each independently, represent a sulfur atom; or a group C-R₃ wherein R₃ represents: a hydrogen atom, a linear or branched C₁-C₂₀, preferably C₁-C₁₂, alkyl group, a cycloalkyl group optionally substituted, an aryl group optionally substituted, a heteroaryl group optionally substituted, a linear or branched C₁-C₂₀, preferably C₁-C₁₂, alkoxyl group, a group -CHO, a carboxyl group -COOR₄ wherein R₄ represents a linear or branched C₁-C₂₀, preferably C₁-C₁₂, alkyl group, an amide group -CONHR₄ or -CON(R₄)₂ wherein R₄ has the meanings indicated above; with the proviso that:
   - one of A, B and C and one of D, E, and F, represents a sulfur atom;
   - E represents a sulfur atom only in the case in which B represents a sulfur atom;
   - when E and B represent a sulfur atom, D represents a group C-R₃ described above, wherein R₃ is different from hydrogen;
   - the sum of the double carbon-carbon bonds (C=C) present in the two thiophene rings and in the benzene ring is equal to 5;
   - R₁ and R₂ each independently represent a hydrogen atom, a linear or branched C₁-C₂₀, preferably C₁-C₁₂, alkyl group, a cycloalkyl group optionally substituted, an aryl group optionally substituted, a heteroaryl group optionally substituted;
said process comprising reacting at least one monohalogenated dithiophene compound having general formula (II): 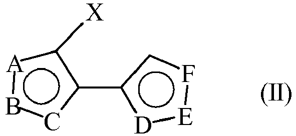 wherein X represents a halogen atom selected from chlorine, bromine, iodine, preferably bromine, A, B, C, D, E and F have the same meanings described above;
with at least one internal alkyne having general formula (III): 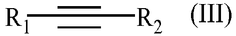 wherein R₁ and R₂ have the same meanings defined above.

For the purposes of the present description and of the following claims, the definitions of the numerical intervals always include the extremes, unless otherwise specified.

The term "C₁-C₂₀ alkyl group" means a linear or branched alkyl group having from 1 to 20 carbon atoms. Specific examples of a C₁-C₂₀ alkyl group are: methyl, ethyl, n-propyl, iso-propyl, n-butyl, iso-butyl, t-butyl, pentyl, ethyl-hexyl, hexyl, heptyl, octyl, nonyl, decyl, dodecyl.

The term "cycloalkyl group" means a cycloalkyl group having from 3 to 10 carbon atoms. Said cycloalkyl group can be optionally substituted by one or more groups, equal to or different from each other, selected from: halogen atoms, such as, for example, fluorine, chlorine, preferably fluorine; hydroxyl groups; C₁-C₂₀ alkyl groups; C₁-C₂₀ alkoxyl groups; cyano groups; amino groups; nitro groups. Specific examples of a cycloalkyl group are: cyclopropyl, 2,2-difluorocyclopropyl, ciclobutyl, ciclopentyl, ciclohexyl, methylcyclohexyl, methoxycyclohexyl, fluorocyclohexyl, phenylcyclohexyl.

The term "aryl group" means an aromatic carbocyclic group. Said aromatic carbocyclic group can be optionally substituted with one or more groups, equal to or different from each other, selected from: halogen atoms such as, for example, fluorine, chlorine, preferably fluorine; hydroxyl groups; C₁-C₂₀ alkyl groups; C₁-C₂₀ alkoxyl groups, cyano groups; amino groups; nitro groups. Specific examples of an aryl group are: phenyl, methylphenyl, trimethylphenyl, methoxyphenyl, hydroxyphenyl, phenyloxyphenyl, fluorophenyl, pentafluorophenyl, chlorophenyl, nitrophenyl, dimethylamminophenyl, naphthyl, phenylnaphthyl, phenanthrene, anthracene.

The term "C₁-C₂₀ alkoxyl group" means a linear or branched alkoxyl group having from 1 to 20 carbon atoms. Specific examples of a C₁-C₂₀ alkoxyl group are: methoxyl, ethoxyl, n-propoxyl, iso-propoxyl, n-butoxyl, iso-butoxyl, t-butoxyl, pentoxyl, hexyloxyl, heptyloxyl, octyloxyl, nonyloxyl, decyloxyl, dodecyloxyl.

The term "heteroaryl group" means an aromatic heterocyclic group, penta- or hexa-atomic, also benzocondensed or heterobicyclic, containing from 1 to 4 heteroatoms selected from nitrogen, oxygen, sulfur, silicon, selenium, phosphorus. Said heteroaryl group can be optionally substituted by one or more groups, equal to or different from each other, selected from: halogen atoms such as, for example, fluorine, chlorine, preferably fluorine; hydroxyl groups; C₁-C₂₀ alkyl groups; C₁-C₂₀ alkoxyl groups; cyano groups; amino groups; nitro groups. Specific examples of a heteroaryl group are: pyridine, methylpyridine, methoxypyridine, phenylpyridine, fluoropyridine, pyrimidine, pyridazine, pyrazine, triazine, tetrazine, quinoline, quinoxaline, quinazoline, furan, thiophene, hexylthiophene, pyrrole, oxazole, thiazole, isooxazole, isothiazole, oxadiazole, thiadiazole, pyrazole, imidazole, triazole, tetrazole, indole, benzofuran, benzothiophene, benzooxazole, benzothiazole, benzooxadiazole, benzothiadiazole, benzopyrazole, benzimidazole, benzotriazole, triazolepyridine, triazolepyrimidine, coumarin.

The above process can be carried out according to the following Scheme 4: 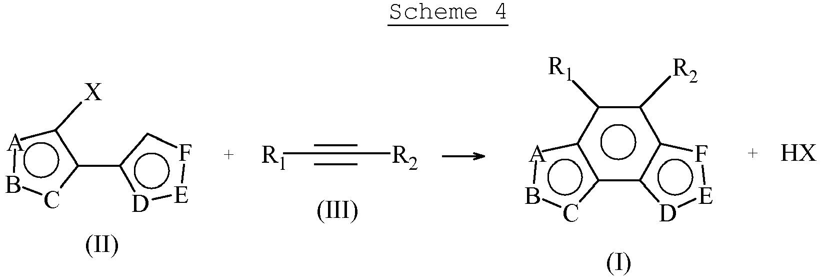 wherein X, A, B, C, D, E, F, R₁ and R₂, have the same meanings described above.

According to a preferred embodiment of the present invention, said monohalogenated dithiophene compound having general formula (II) and said internal alkyne having general formula (III) can be used in molar ratios ranging from 1:2 to 1:10, preferably ranging from 1:2 to 1:5.

According to a preferred embodiment of the present invention, said process relates to the preparation of 4,5-dipropylbenzo[2,1-*b*:3,4-*b*']dithiophene corresponding to a benzodithiophene compound having general formula (I) wherein C and D represent a sulfur atom, A, B, E and F represent a group C-R₃ wherein R₃ represents a hydrogen atom, and R₁ and R₂ represent an n-propyl group, said process comprising reacting 3-bromo-2,2'-dithiophene corresponding to a monohalogenated dithiophene compound having general formula (II) wherein X represents a bromine atom, C and D represent a sulfur atom and A, B, E and F represent a group C-R₃ wherein R₃ represents a hydrogen atom, with 4-octyne corresponding to an internal alkyne having general formula (III) wherein R₁ and R₂ represent an n-propyl group.

According to a further preferred embodiment of the present invention, said process relates to the preparation of 4,5-dipropylbenzo[1,2-*b*:4,3-*b*']dithiophene corresponding to a benzodithiophene compound having general formula (I) wherein A and F represent a sulfur atom, B, C, D and E represent a group C-R₃ wherein R₃ represents a hydrogen atom, and R₁ and R₂ represent an n-propyl group, said process comprising reacting 3-bromo-2,2'-dithiophene corresponding to a monohalogenated dithiophene compound having general formula (II) wherein X represents a bromine atom, A and F represent a sulfur atom and B, C, D and E represent a group C-R₃ wherein R₃ represents a hydrogen atom, with 4-octyne corresponding to an internal alkyne having general formula (III) wherein R₁ and R₂ represent an n-propyl group.

According to a preferred embodiment of the present invention, said process can be carried out in the presence of at least one weak organic base.

According to a preferred embodiment of the present invention, said weak organic base can be selected, for example, from: carboxylates of alkaline (e.g., sodium, potassium, caesium) or alkaline-earth (e.g., magnesium, calcium) metals such as, for example, potassium acetate, sodium acetate, caesium acetate, magnesium acetate, calcium acetate, potassium propionate, sodium propionate, caesium propionate, magnesium propionate, calcium propionate, or mixtures thereof; carbonates of alkaline (e.g., lithium, sodium, potassium, caesium) or alkaline-earth (e.g., magnesium, calcium) metals such as, for example, lithium carbonate, potassium carbonate, sodium carbonate, caesium carbonate, magnesium carbonate, calcium carbonate, or mixtures thereof; bicarbonates of alkaline (e.g., lithium, sodium, potassium, caesium) or alkaline-earth (e.g., magnesium, calcium) metals such as, for example, lithium bicarbonate, potassium bicarbonate, sodium bicarbonate, caesium bicarbonate, magnesium bicarbonate, calcium bicarbonate, or mixtures thereof; or mixtures thereof. Said weak organic base is preferably selected from potassium acetate, potassium carbonate.

According to a preferred embodiment of the present invention, said monohalogenated dithiophene compound having general formula (II) and said weak organic base can be used in molar ratios ranging from 1:2.2 to 1:20, preferably ranging from 1:2.5 to 1:4.

According to a preferred embodiment of the present invention, said process can be carried out in the presence of at least one catalyst containing palladium.

According to a preferred embodiment of the present invention, said catalyst containing palladium can be selected from: compounds of palladium in oxidation state (0) or (II) such as, for example, palladium(II)chloride [PdCl₂], palladium(II) acetate [Pd(OAc)₂], bis(dibenzylidene)palladium(0) [Pd₂(dba)₃ wherein dba = C₆H₅CH=CHCOCH=CHC₆H₅], bis(acetonitrile)-palladium(II) chloride [Pd(CH₃CN)₂Cl₂], bis(triphenylphosphine)palladium(II) chloride [Pd(PPh₃)₂Cl₂], bis(triphenylphosphine)palladium(II) acetate [Pd(PPh₃)₂(OAc)₂], tetrakis-(triphenylphosphine)-palladium(0) [Pd(PPh₃)₄], or mixtures thereof. Said catalyst containing palladium is preferably selected from palladium(II) acetate [Pd(OAc)₂], bis(triphenylphosphine)palladium(II) chloride [Pd(PPh₃)₂Cl₂].

According to a preferred embodiment of the present invention, said monohalogenated dithiophene compound having general formula (II) and said catalyst containing palladium can be used in molar ratios ranging from 100:0.1 to 100:8, preferably ranging from 100:0.4 to 100:6.

According to a preferred embodiment of the present invention, said monohalogenated dithiophene compound having general formula (II) can be used in a molar concentration ranging from 0.05 mmoles to 2 mmoles, preferably ranging from 0.1 mmoles to 1.5 mmoles.

According to a preferred embodiment of the present invention, said process can be carried out in the presence of at least one dipolar aprotic organic solvent.

According to a preferred embodiment of the present invention, said dipolar aprotic organic solvent can be selected from N,N-dimethylacetamide (DMAc), dimethylsulfoxide (DMSO), N-methylpyrrolidone (NMP), N,N-dimethylformamide (DMF), or mixtures thereof. Said dipolar aprotic organic solvent is preferably selected from N,N-dimethylacetamide (DMAc), N,N-dimethylformamide (DMF).

According to a preferred embodiment of the present invention, said process can be carried out in the presence of at least one quaternary ammonium salt such as, for example, a tetraalkylammonium bromide, preferably tetrabutylammonium bromide.

According to a preferred embodiment of the present invention, said monohalogenated dithiophene compound having general formula (II) and said quaternary ammonium salt can be used in molar ratios ranging from 1:0.5 to 1:5, preferably ranging from 1:0.8 to 1:2.

According to a further preferred embodiment of the present invention, said process can be carried out in the presence of at least one lithium salt such as, for example, lithium bromide, lithium chloride, preferably lithium bromide.

According to a preferred embodiment of the present invention, said monohalogenated dithiophene compound having general formula (II) and said lithium salt can be used in molar ratios ranging from 1:0.5 to 1:5, preferably ranging from 1:0.8 to 1:4.

It should be observed that for the purposes of the process object of the present invention, if palladium(II) acetate [Pd(OAc)₂] is used as catalyst, it is preferable to use tetra-alkylammonium bromide whereas, if bis(tri-phenylphosphine)palladium(II) chloride [Pd(PPh₃)₂Cl₂] is used as catalyst, it is preferable to use a lithium salt.

According to a preferred embodiment of the present invention, said process can be carried out at a temperature ranging from 80°C to 170°C, preferably ranging from 100°C to 150°C.

According to a preferred embodiment of the present invention, said process can be carried out for a time ranging from 30 minutes to 72 hours, preferably ranging from 3 hours to 48 hours.

The monohalogenated dithiophene compound having general formula (II) can be obtained according to processes known in the art, such as, for example, by halogenation of the corresponding dithiophene compounds, or through coupling reactions catalyzed by copper compounds. Greater details relating to these processes can be found, for example, in the article of Xie L.-H. et al.: "An Effective Strategy to Tune Supramolecular Interaction via a Spiro-Bridged Spacer in Oligothiophene-S,S-dioxides and Their Anomalous Photoluminescent Behavior","Organic Letters" (2007), Vol. 9, No. 9, pages 1619-1622; or in the article of Ogawa C. et al.: "A Simple and Efficient Route to N-Functionalized Dithieno[3,2-b:2',3'-d]pyrroles: Fused-Ring Building Blocks for New Conjugated Polymeric Systems", "Journal of Organic Chemistry" (2003), Vol. 68 (7), pages 2921-2928.

The internal alkyne having general formula (III) can be prepared according to processes known in the art, for example, by nucleophilic substitution of an alkyl acetylide on an alkyl halide as described, for example, in the article of Kirkham J. E. D. et al.: "Asymmetric synthesis of cytotoxic sponge metabolites R-strongylodiols A and B", "Tetrahedron Letters" (2004), Vol. 45, No. 29, pages 5645-5648; or can be available on the market.

Some illustrative and non-limiting examples are provided for a better understanding of the present invention and for its practical embodiment.

### EXAMPLE 1

### Preparation of 4,5-dipropylbenzo[2,1-b:3,4-b']dithiophene having formula (a)

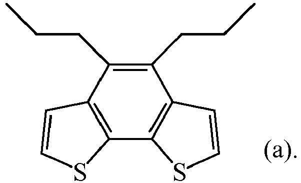

The following products were charged in order into a pyrex glass reactor equipped with a screw stopper: 573 mg of potassium carbonate (4.15 mmoles), 445 mg of tetrabutylammonium bromide (1.38 mmoles), 15 mg of palladium(II)acetate [Pd(OAc)₂] (0.069 mmoles), 341 mg of 3-bromo-2,2'-dithiophene (1.4 mmoles) dissolved in 5 ml of N,N-dimethylacetamide and finally 455 mg of 4-octyne (4.14 mmoles). After closing the reactor, it was placed in an oil bath preheated to 130°C, for 16 hours. After cooling to room temperature (25°C), a saturated aqueous solution of sodium chloride (50 ml) was added to the reaction mixture and the whole mixture was extracted with ethyl acetate (3 x 25 ml). The organic phase obtained was washed to neutrality with water (3 x 25 ml) and subsequently anhydrified on sodium sulfate and evaporated. The residue obtained was purified by elution on a silica gel chromatographic column (eluent: heptane), obtaining 308 mg of 4,5-dipropylbenzo[2,1-b:3,4-*b*']dithiophene as a white solid (yield 80%).

### EXAMPLE 2

### Preparation of 4,5-dipropylbenzo[1,2-b:4,3-b']dithiophene having formula (b)

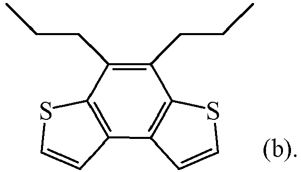

The following products were charged in order into a pyrex glass reactor equipped with a screw stopper: 206 mg of potassium carbonate (1.5 mmoles), 43 mg of lithium bromide (0.5 mmoles), 17 mg of bis(triphenylphosphine)palladium(II) chloride [Pd(PPh₃)₂Cl₂] (0.0248 mmoles), 123 mg of 2-bromo-3,3'-dithiophene (0.5 mmoles) dissolved in 4 ml of N,N-dimethylformamide and finally 165 mg of 4-octyne (1.5 mmoles). After closing the reactor, it was placed in an oil bath preheated to 130°C, for 48 hours. After cooling to room temperature (25°C), a saturated aqueous solution of sodium chloride (50 ml) was added to the reaction mixture and the whole mixture was extracted with ethyl acetate (3 x 25 ml). The organic phase obtained was washed to neutrality with water (3 x 25 ml) and subsequently anhydrified on sodium sulfate and evaporated. The residue obtained was purified by means of elution on a silica gel chromatographic column (eluent: heptane), obtaining 110 mg of 4,5-dipropylbenzo[1,2-*b*:4,3-*b*']dithiophene as a white solid (yield 80%).

## Claims

1. A process for the preparation of a benzodithiophene compound having general formula (I) : 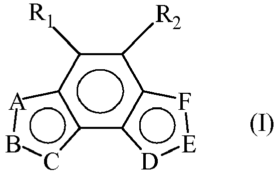 wherein:
- A, B, C, D, E and F, each independently, represent a sulfur atom; or a group C-R₃ wherein R₃ represents: a hydrogen atom, a linear or branched C₁-C₂₀ alkyl group, a cycloalkyl group optionally substituted, an aryl group optionally substituted, a heteroaryl group optionally substituted, a linear or branched C₁-C₂₀ alkoxyl group, a group -CHO, a carboxyl group -COOR₄ wherein R₄ represents a linear or branched C₁-C₂₀ alkyl group, an amide group -CONHR₄ or -CON(R₄)₂ wherein R₄ has the meanings indicated above; with the proviso that:
- one of A, B and C and one of D, E, and F, represents a sulfur atom;
- E represents a sulfur atom only in the case in which B represents a sulfur atom;
- when E and B represent a sulfur atom, D represents a group C-R₃ described above, wherein R₃ is different from hydrogen;
- the sum of the double carbon-carbon bonds (C=C) present in the two thiophene rings and in the benzene ring is equal to 5;
- R₁ and R₂ each independently represent a hydrogen atom, a linear or branched C₁-C₂₀ alkyl group, a cycloalkyl group optionally substituted, an aryl group optionally substituted, a heteroaryl group optionally substituted;
said process comprising reacting at least one monohalogenated dithiophene compound having general formula (II): 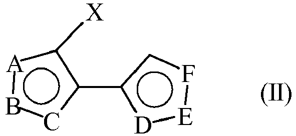 wherein X represents a halogen atom selected from chlorine, bromine, iodine, A, B, C, D, E and F have the same meanings described above:
with at least one internal alkyne having general formula (III): 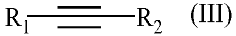 wherein R₁ and R₂ have the same meanings defined above.

2. The process according to claim 1, wherein said monohalogenated dithiophene compound having general formula (II) and said internal alkyne having general formula (III) are used in molar ratios ranging from 1:2 to 1:10.

3. The process according to claim 1 or 2, wherein said process relates to the preparation of 4,5-dipropylbenzo[2,1-*b*:3,4-*b'*]dithiophene corresponding to a benzodithiophene compound having general formula (I) wherein C and D represent a sulfur atom, A, B, E and F represent a group C-R₃ wherein R₃ represents a hydrogen atom, and R₁ and R₂ represent an n-propyl group, said process comprising reacting 3-bromo-2,2'-dithiophene corresponding to a monohalogenated dithiophene compound having general formula (II) wherein X represents a bromine atom, C and D represent a sulfur atom and A, B, E and F represent a group C-R₃ wherein R₃ represents a hydrogen atom, with 4-octyne corresponding to an internal alkyne having general formula (III) wherein R₁ and R₂ represent an n-propyl group.

4. The process according to claim 1 or 2, wherein said process relates to the preparation of 4,5-dipropylbenzo[1,2-*b*:4,3-*b*']dithiophene corresponding to a benzodithiophene compound having general formula (I) wherein A and F represent a sulfur atom, B, C, D and E represent a group C-R₃ wherein R₃ represents a hydrogen atom, and R₁ and R₂ represent an n-propyl group, said process comprising reacting 3-bromo-2,2'-dithiophene corresponding to a monohalogenated dithiophene compound having general formula (II) wherein X represents a bromine atom, A and F represent a sulfur atom and B, C, D and E represent a group C-R₃ wherein R₃ represents a hydrogen atom, with 4-octyne corresponding to an internal alkyne having general formula (III) wherein R₁ and R₂ represent an n-propyl group.

5. The process according to any of the previous claims, wherein said process is carried out in the presence of at least one weak organic base.

6. The process according to claim 5, wherein said weak organic base is selected from: carboxylates of alkaline or alkaline-earth metals such as potassium acetate, sodium acetate, caesium acetate, magnesium acetate, calcium acetate, potassium propionate, sodium propionate, caesium propionate, magnesium propionate, calcium propionate, or mixtures thereof; carbonates of alkaline or alkaline-earth metals such as lithium carbonate, potassium carbonate, sodium carbonate, caesium carbonate, magnesium carbonate, calcium carbonate, or mixtures thereof; bicarbonates of alkaline or alkaline-earth metals such as lithium bicarbonate, potassium bicarbonate, sodium bicarbonate, caesium bicarbonate, magnesium bicarbonate, calcium bicarbonate, or mixtures thereof; or mixtures thereof.

7. The process according to any of the previous claims, wherein said monohalogenated dithiophene compound having general formula (II) and said weak organic base are used in molar ratios ranging from 1:2.2 to 1:20.

8. The process according to any of the previous claims, wherein said process is carried out in the presence of at least one catalyst containing palladium.

9. The process according to claim 8, wherein said catalyst containing palladium is selected from: compounds of palladium in oxidation state (0) or (II) such as palladium(II)chloride [PdCl₂], palladium(II)acetate [Pd(OAc)₂], bis(dibenzyl-idene)palladium(0) [Pd₂(dba)₃ wherein dba = C₆H₅CH=CHCOCH=CHC₆H₅], bis(acetonitrile)-palladium(II)chloride [Pd(CH₃CN)₂Cl₂], bis(triphenylphosphine)palladium(II)chloride [Pd(PPh₃)₂Cl₂], bis(triphenylphosphine)-palladium(II)acetate [Pd(PPh₃)₂(OAc)₂], tetrakis-(triphenylphosphine)palladium(0) [Pd(PPh₃)₄], or mixtures thereof.

10. The process according to any of the previous claims, wherein said monohalogenated dithiophene compound having general formula (II) and said catalyst containing palladium are used in molar ratios ranging from 100:0.1 to 100:8.

11. The process according to any of the previous claims, wherein said monohalogenated dithiophene compound having general formula (II) is used in a molar concentration ranging from 0.05 mmoles to 2 mmoles.

12. The process according to any of the previous claims, wherein said process is carried out in the presence of at least one dipolar aprotic organic solvent.

13. The process according to claim 12, wherein said dipolar aprotic organic solvent is selected from N,N-dimethylacetamide (DMAc), dimethylsulfoxide (DMSO), N-methylpyrrolidone (NMP), N,N-dimethylformamide (DMF), or mixtures thereof.

14. The process according to any of the previous claims, wherein said process is carried out in the presence of at least one quaternary ammonium salt such as a tetraalkylammonium bromide.

15. The process according to claim 14, wherein said monohalogenated dithiophene compound having general formula (II) and said quaternary ammonium salt are used in molar ratios ranging from 1:0.5 to 1:5.

16. The process according to any of the previous claims, wherein said process is carried out in the presence of at least one lithium salt such as lithium bromide, lithium chloride.

17. The process according to claim 16, wherein said monohalogenated dithiophene compound having general formula (II) and said lithium salt are used in molar ratios ranging from 1:0.5 to 1:5.

18. The process according to any of the previous claims, wherein said process is carried out at a temperature ranging from 80°C to 170°C.

19. The process according to any of the previous claims, wherein said process is carried out for a time ranging from 30 minutes to 72 hours.

## Patentansprüche

1. Ein Verfahren zur Herstellung einer Benzodithiophenverbindung mit der allgemeinen Formel (I): 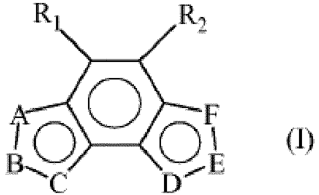 wobei:
- A, B, C, D, E und F jeweils unabhängig voneinander ein Schwefelatom oder eine C-R₃-Gruppe darstellen, wobei R₃ Folgendes darstellt: ein Wasserstoffatom, eine lineare oder verzweigte C₁-C₂₀-Alkylgruppe, eine optional substituierte Cycloalkylgruppe, eine optional substituierte Arylgruppe, eine optional substituierte Heteroarylgruppe, eine lineare oder verzweigte C₁-C₂₀-Alkoxylgruppe, eine -CHO-Gruppe, eine -COOR₄-Carboxylgruppe, wobei R₄ eine lineare oder verzweigte C₁-C₂₀-Alkylgruppe, eine -CONHR₄- oder -CON(R₄)₂-Amidgruppe darstellt, wobei R₄ die oben angegebenen Bedeutungen aufweist; mit der Maßgabe, dass:
- eines von A, B und C und eines von D, E und F ein Schwefelatom darstellen;
- E nur in dem Fall ein Schwefelatom darstellt, dass B ein Schwefelatom darstellt;
- wenn E und B ein Schwefelatom darstellen, D eine wie oben beschriebene C-R₃-Gruppe darstellt, wobei R₃ kein Wasserstoff ist;
- die Summe der Kohlenstoff-Kohlenstoff-Doppelbindungen (C=C), die in den zwei Thiophenringen und in dem Benzolring vorhanden sind, gleich 5 ist;
- R₁ und R₂ jeweils unabhängig voneinander ein Wasserstoffatom, eine lineare oder verzweigte C₁-C₂₀-Alkylgruppe, eine optional substituierte Cycloalkylgruppe, eine optional substituierte Arylgruppe, eine optional substituierte Heteroarylgruppe darstellen;
wobei das Verfahren das Reagierenlassen mindestens einer monohalogenierten Dithiophenverbindung mit der allgemeinen Formel (II) beinhaltet: 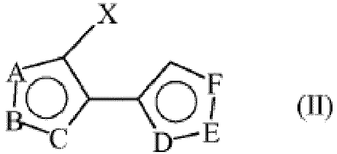
wobei X ein Halogenatom darstellt, ausgewählt aus Chlor, Brom, Iod, A, B, C, D, E und
F mit den oben beschriebenen Bedeutungen:
mit mindestens einem internen Alkin mit der allgemeinen Formel (III): 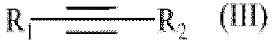 wobei R₁ und R₂ die gleichen Bedeutungen wie oben definiert aufweisen.

2. Verfahren gemäß Anspruch 1, wobei die monohalogenierte Dithiophenverbindung mit der allgemeinen Formel (II) und das interne Alkin mit der allgemeinen Formel (III) in molaren Verhältnissen im Bereich von 1 : 2 bis 1 : 10 verwendet werden.

3. Verfahren gemäß Anspruch 1 oder 2, wobei das Verfahren die Herstellung von 4,5-Dipropylbenzo[2,1-*b*:3,4-*b*']dithiophen entsprechend einer Benzodithiophenverbindung mit der allgemeinen Formel (I) betrifft, wobei C und D ein Schwefelatom darstellen, A, B, E und F eine C-R₃-Gruppe darstellen, wobei R₃ ein Wasserstoffatom darstellt und R₁ und R₂ eine n-Propylgruppe darstellen, wobei das Verfahren das Reagierenlassen von 3-Brom-2,2'-dithiophen entsprechend einer monohalogenierten Dithiophenverbindung mit der allgemeinen Formel (II) beinhaltet, wobei X ein Bromatom darstellt, C und D ein Schwefelatom darstellen und A, B, E und F eine C-R₃-Gruppe darstellen, wobei R₃ ein Wasserstoffatom darstellt, wobei 4-Octin einem internen Alkin mit der allgemeinen Formel (III) entspricht, wobei R₁ und R₂ eine n-Propylgruppe darstellen.

4. Verfahren gemäß Anspruch 1 oder 2, wobei das Verfahren die Herstellung von 4,5-Dipropylbenzo[1,2-*b*:4,3-*b*']dithiophen entsprechend einer Benzodithiophenverbindung mit der allgemeinen Formel (I) betrifft, wobei A und F ein Schwefelatom darstellen, B, C, D und E eine C-R₃-Gruppe darstellen, wobei R₃ ein Wasserstoffatom darstellt und R₁ und R₂ eine n-Propylgruppe darstellen, wobei das Verfahren das Reagierenlassen von 3-Brom-2,2'-dithiophen entsprechend einer monohalogenierten Dithiophenverbindung mit der allgemeinen Formel (II) beinhaltet, wobei X ein Bromatom darstellt, A und F ein Schwefelatom darstellen und B, C, D und E eine C-R₃-Gruppe darstellen, wobei R₃ ein Wasserstoffatom darstellt, wobei 4-Octin einem internen Alkin mit der allgemeinen Formel (III) entspricht, wobei R₁ und R₂ eine n-Propylgruppe darstellen.

5. Verfahren gemäß einem der vorhergehenden Ansprüche, wobei das Verfahren in Gegenwart von mindestens einer schwachen organischen Base durchgeführt wird.

6. Verfahren gemäß Anspruch 5, wobei die schwache organische Base aus Folgendem ausgewählt ist: Carboxylaten von Alkali- oder Erdalkalimetallen, wie etwa Kaliumacetat, Natriumacetat, Caesiumacetat, Magnesiumacetat, Calciumacetat, Kaliumpropionat, Natriumpropionat, Caesiumpropionat, Magnesiumpropionat, Calciumpropionat oder Mischungen davon; Carbonaten von Alkali- oder Erdalkalimetallen, wie etwa Lithiumcarbonat, Kaliumcarbonat, Natriumcarbonat, Caesiumcarbonat, Magnesiumcarbonat, Calciumcarbonat oder Mischungen davon; Bicarbonaten von Alkali- oder Erdalkalimetallen, wie etwa Lithiumbicarbonat, Kaliumbicarbonat, Natriumbicarbonat, Caesiumbicarbonat, Magnesiumbicarbonat, Calciumbicarbonat oder Mischungen davon; oder Mischungen davon.

7. Verfahren gemäß einem der vorhergehenden Ansprüche, wobei die monohalogenierte Dithiophenverbindung mit der allgemeinen Formel (II) und die schwache organische Base in molaren Verhältnissen im Bereich von 1 : 2,2 bis 1 : 20 verwendet werden.

8. Verfahren gemäß einem der vorhergehenden Ansprüche, wobei das Verfahren in Gegenwart von mindestens einem Palladium enthaltenden Katalysator durchgeführt wird.

9. Verfahren gemäß Anspruch 8, wobei der Palladium enthaltende Katalysator aus Folgendem ausgewählt ist: Verbindungen von Palladium in der Oxidationsstufe (0) oder (II), wie etwa Palladium(II)chlorid [PdCl₂], Palladium(II)acetat [Pd(OAc)₂], Bis(dibenzyliden)palladium(0) [Pd₂(dba)₃, wobei dba = C₆H₅CH=CHCOCH=CHC₆H₅], Bis(acetonitril)palladium(II)chlorid [Pd(CH₃CN)₂Cl₂], Bis(triphenylphosphin)palladium(II)chlorid [Pd(PPh₃)₂Cl₂], Bis(triphenylphosphin)palladium(II)acetat [Pd(PPh₃)₂(OAc)₂], Tetrakis(triphenylphosphin)palladium(0) [Pd(PPh₃)₄] oder Mischungen davon.

10. Verfahren gemäß einem der vorhergehenden Ansprüche, wobei die monohalogenierte Dithiophenverbindung mit der allgemeinen Formel (II) und der Palladium enthaltende Katalysator in molaren Verhältnissen im Bereich von 100 : 0,1 bis 100 : 8 verwendet werden.

11. Verfahren gemäß einem der vorhergehenden Ansprüche, wobei die monohalogenierte Dithiophenverbindung mit der allgemeinen Formel (II) in einer molaren Konzentration im Bereich von 0,05 mmol bis 2 mmol verwendet wird.

12. Verfahren gemäß einem der vorhergehenden Ansprüche, wobei das Verfahren in Gegenwart von mindestens einem dipolar aprotischen organischen Lösungsmittel durchgeführt wird.

13. Verfahren gemäß Anspruch 12, wobei das dipolare aprotische organische Lösungsmittel ausgewählt ist aus N,N-Dimethylacetamid (DMAc), Dimethylsulfoxid (DMSO), N-Methylpyrrolidon (NMP), N,N-Dimethylformamid (DMF) oder Mischungen davon.

14. Verfahren gemäß einem der vorhergehenden Ansprüche, wobei das Verfahren in Gegenwart von mindestens einem quartären Ammoniumsalz, wie etwa einem Tetraalkylammoniumbromid, durchgeführt wird.

15. Verfahren gemäß Anspruch 14, wobei die monohalogenierte Dithiophenverbindung mit der allgemeinen Formel (II) und das quartäre Ammoniumsalz in molaren Verhältnissen im Bereich von 1 : 0,5 bis 1 : 5 verwendet werden.

16. Verfahren gemäß einem der vorhergehenden Ansprüche, wobei das Verfahren in Gegenwart von mindestens einem Lithiumsalz, wie etwa Lithiumbromid, Lithiumchlorid, durchgeführt wird.

17. Verfahren gemäß Anspruch 16, wobei die monohalogenierte Dithiophenverbindung mit der allgemeinen Formel (II) und das Lithiumsalz in molaren Verhältnissen im Bereich von 1 : 0,5 bis 1 : 5 verwendet werden.

18. Verfahren gemäß einem der vorhergehenden Ansprüche, wobei das Verfahren bei einer Temperatur im Bereich von 80 °C bis 170 °C durchgeführt wird.

19. Verfahren gemäß einem der vorhergehenden Ansprüche, wobei das Verfahren für eine Zeitperiode im Bereich von 30 Minuten bis 72 Stunden durchgeführt wird.

## Revendications

1. Un procédé pour la préparation d'un composé benzodithiophénique ayant la formule générale (I) : 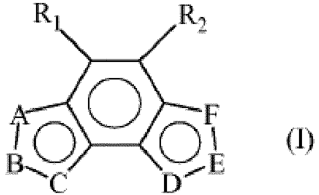 où :
- A, B, C, D, E et F, chacun indépendamment, représentent un atome de soufre ; ou un groupe C-R₃ dans lequel R₃ représente : un atome d'hydrogène, un groupe alkyle en C₁-C₂₀ linéaire ou ramifié, un groupe cycloalkyle facultativement substitué, un groupe aryle facultativement substitué, un groupe hétéroaryle facultativement substitué, un groupe alcoxyle en C₁-C₂₀ linéaire ou ramifié, un groupe -CHO, un groupe carboxyle -COOR₄ dans lequel R₄ représente un groupe alkyle en C₁-C₂₀ linéaire ou ramifié, un groupe amide -CONHR₄ ou -CON(R₄)₂ dans lequel R₄ a les significations indiquées ci-dessus ; à la condition que :
- l'un d'entre A, B et C et l'un d'entre D, E, et F, représente un atome de soufre ;
- E représente un atome de soufre seulement dans le cas où B représente un atome de soufre ;
- lorsque E et B représentent un atome de soufre, D représente un groupe C-R₃ décrit ci-dessus, dans lequel R₃ est différent de l'hydrogène ;
- la somme des doubles liaisons carbone-carbone (C=C) présentes dans les deux cycles thiophéniques et dans le cycle benzénique est égale à 5 ;
- R₁ et R₂ représentent chacun indépendamment un atome d'hydrogène, un groupe alkyle en C₁-C₂₀ linéaire ou ramifié, un groupe cycloalkyle facultativement substitué, un groupe aryle facultativement substitué, un groupe hétéroaryle facultativement substitué ;
ledit procédé comprenant le fait de faire réagir au moins un composé dithiophénique monohalogéné ayant la formule générale (II) : 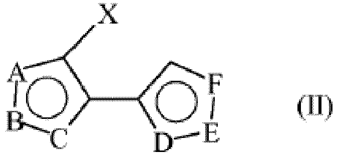 où X représente un atome d'halogène sélectionné parmi le chlore, le brome, l'iode, A, B, C, D, E et F ont les mêmes significations décrites ci-dessus :
avec au moins un alcyne interne ayant la formule générale (III) : 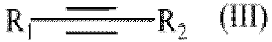 où R₁ et R₂ ont les mêmes significations définies ci-dessus.

2. Le procédé selon la revendication 1, où ledit composé dithiophénique monohalogéné ayant la formule générale (II) et ledit alcyne interne ayant la formule générale (III) sont utilisés dans des rapports molaires compris dans la gamme allant de 1/2 à 1/10.

3. Le procédé selon la revendication 1 ou la revendication 2, où ledit procédé porte sur la préparation de 4,5-dipropylbenzo[2,1-*b*:3,4-*b*']dithiophène correspondant à un composé benzodithiophénique ayant la formule générale (I) où C et D représentent un atome de soufre, A, B, E et F représentent un groupe C-R₃ dans lequel R₃ représente un atome d'hydrogène, et R₁ et R₂ représentent un groupe n-propyle, ledit procédé comprenant le fait de faire réagir du 3-bromo-2,2'-dithiophène correspondant à un composé dithiophénique monohalogéné ayant la formule générale (II) où X représente un atome de brome, C et D représentent un atome de soufre et A, B, E et F représentent un groupe C-R₃ dans lequel R₃ représente un atome d'hydrogène, avec du 4-octyne correspondant à un alcyne interne ayant la formule générale (III) où R₁ et R₂ représentent un groupe n-propyle.

4. Le procédé selon la revendication 1 ou la revendication 2, où ledit procédé porte sur la préparation de 4,5-dipropylbenzo[1,2-*b*:4,3-*b*']dithiophène correspondant à un composé benzodithiophénique ayant la formule générale (I) où A et F représentent un atome de soufre, B, C, D et E représentent un groupe C-R₃ dans lequel R₃ représente un atome d'hydrogène, et R₁ et R₂ représentent un groupe n-propyle, ledit procédé comprenant le fait de faire réagir du 3-bromo-2,2'-dithiophène correspondant à un composé dithiophénique monohalogéné ayant la formule générale (II) où X représente un atome de brome, A et F représentent un atome de soufre et B, C, D et E représentent un groupe C-R₃ dans lequel R₃ représente un atome d'hydrogène, avec du 4-octyne correspondant à un alcyne interne ayant la formule générale (III) où R₁ et R₂ représentent un groupe n-propyle.

5. Le procédé selon n'importe lesquelles des revendications précédentes, où ledit procédé est mis en oeuvre en présence d'au moins une base organique faible.

6. Le procédé selon la revendication 5, où ladite base organique faible est sélectionnée parmi : des carboxylates de métaux alcalins ou alcalino-terreux tels que de l'acétate de potassium, de l'acétate de sodium, de l'acétate de césium, de l'acétate de magnésium, de l'acétate de calcium, du propionate de potassium, du propionate de sodium, du propionate de césium, du propionate de magnésium, du propionate de calcium, ou des mélanges de ceux-ci ; des carbonates de métaux alcalins ou alcalino-terreux tels que du carbonate de lithium, du carbonate de potassium, du carbonate de sodium, du carbonate de césium, du carbonate de magnésium, du carbonate de calcium, ou des mélanges de ceux-ci ; des bicarbonates de métaux alcalins ou alcalino-terreux tels que du bicarbonate de lithium, du bicarbonate de potassium, du bicarbonate de sodium, du bicarbonate de césium, du bicarbonate de magnésium, du bicarbonate de calcium, ou des mélanges de ceux-ci ; ou des mélanges de ceux-ci.

7. Le procédé selon n'importe lesquelles des revendications précédentes, où ledit composé dithiophénique monohalogéné ayant la formule générale (II) et ladite base organique faible sont utilisés dans des rapports molaires compris dans la gamme allant de 1/2,2 à 1/20.

8. Le procédé selon n'importe lesquelles des revendications précédentes, où ledit procédé est mis en oeuvre en présence d'au moins un catalyseur contenant du palladium.

9. Le procédé selon la revendication 8, où ledit catalyseur contenant du palladium est sélectionné parmi : des composés de palladium à l'état d'oxydation (0) ou (II) tels que le chlorure de palladium (II) [PdCl₂], l'acétate de palladium (II) [Pd(OAc)₂], le bis(dibenzylidène)palladium (0) [Pd₂(dba)₃ où dba = C₆H₅CH=CHCOCH=CHC₆H₅], le chlorure de bis(acétonitrile)palladium (II) [Pd(CH₃CN)₂Cl₂], le chlorure de bis(triphénylphosphine)palladium (II) [Pd(PPh₃)₂Cl₂], l'acétate de bis(triphénylphosphine)palladium (II) [Pd(PPh₃)₂(OAc)₂], le tétrakis(triphénylphosphine)palladium (0) [Pd(PPh₃)₄], ou des mélanges de ceux-ci.

10. Le procédé selon n'importe lesquelles des revendications précédentes, où ledit composé dithiophénique monohalogéné ayant la formule générale (II) et ledit catalyseur contenant du palladium sont utilisés dans des rapports molaires compris dans la gamme allant de 100/0,1 à 100/8.

11. Le procédé selon n'importe lesquelles des revendications précédentes, où ledit composé dithiophénique monohalogéné ayant la formule générale (II) est utilisé dans une concentration molaire comprise dans la gamme allant de 0,05 mmole à 2 mmoles.

12. Le procédé selon n'importe lesquelles des revendications précédentes, où ledit procédé est mis en oeuvre en présence d'au moins un solvant organique aprotique dipolaire.

13. Le procédé selon la revendication 12, où ledit solvant organique aprotique dipolaire est sélectionné parmi le N,N-diméthylacétamide (DMAc), le diméthylsulfoxyde (DMSO), la N-méthylpyrrolidone (NMP), le N,N-diméthylformamide (DMF), ou des mélanges de ceux-ci.

14. Le procédé selon n'importe lesquelles des revendications précédentes, où ledit procédé est mis en oeuvre en présence d'au moins un sel d'ammonium quaternaire tel qu'un bromure de tétraalkylammonium.

15. Le procédé selon la revendication 14, où ledit composé dithiophénique monohalogéné ayant la formule générale (II) et ledit sel d'ammonium quaternaire sont utilisés dans des rapports molaires compris dans la gamme allant de 1/0,5 à 1/5.

16. Le procédé selon n'importe lesquelles des revendications précédentes, où ledit procédé est mis en oeuvre en présence d'au moins un sel de lithium tel que du bromure de lithium, du chlorure de lithium.

17. Le procédé selon la revendication 16, où ledit composé dithiophénique monohalogéné ayant la formule générale (II) et ledit sel de lithium sont utilisés dans des rapports molaires compris dans la gamme allant de 1/0,5 à 1/5.

18. Le procédé selon n'importe lesquelles des revendications précédentes, où ledit procédé est mis en oeuvre à une température comprise dans la gamme allant de 80 °C à 170 °C.

19. Le procédé selon n'importe lesquelles des revendications précédentes, où ledit procédé est mis en oeuvre pour une durée comprise dans la gamme allant de 30 minutes à 72 heures.
